# FASCICULE DE BREVET EUROPEEN

(11) **EP 2 037 834 B1**
(45) Date de publication et mention de la délivrance du brevet: **14.09.2016**
(21) Numéro de dépôt: 07787233.1
(22) Date de dépôt: 09.07.2007
(51) Int. Cl.: A61C 13/00, A61C 8/00, A61C 1/08

(54) **PROCÉDÉ DE FABRICATION D'UNE PROTHÈSE OSSEUSE OU D'UNE SIMULATION PREIMPLANTAIRE ET APPAREILLAGE MIS EN OEUVRE**
HERSTELLUNGSVERFAHREN EINER KNOCHENPROTHESE ODER EINER PREIMPLANTAIRE SIMULATION UND VORRICHTUNG ZUM BETREIBEN DIESES VERFAHRENS
METHOD FOR PRODUCING A BONE PROSTHESIS OR PREIMPLANTING SIMULATION AND DEVICE FOR APPLYING THIS METHOD

(30) Priorité: 11.07.2006 EP 06116963
(43) Date de publication de la demande: 25.03.2009
(73) Titulaire: 2INGIS S.A., 1120 Bruxelles (BE)
(72) Inventeur: DE MOYER, Philippe, Albert, Paul, Ghislain, 1650 Beersel (BE)
(74) Mandataire: Farmer, Guy Dominic
(86) Numéro de dépôt international: PCT/EP2007/056957
(87) Numéro de publication internationale: WO 2008/006802

(56) Documents cités:
- EP-A- 0 930 050
- EP-A- 1 547 544
- EP-A2- 0 328 911
- WO-A-00/74585
- WO-A-98/43528
- WO-A-2004/075771
- WO-A-2007/085719
- WO-A1-2006/082198
- DE-U1- 29 705 088
- US-A- 2 621 408
- US-A- 5 207 753
- US-A- 5 954 769

## Description

La présente invention est relative à un procédé de fabrication d'une prothèse osseuse à implanter dans un os d'un patient, et à un appareillage mis en oeuvre pour une telle implantation.

Le procédé et l'appareillage suivant l'invention sont destinés plus particulièrement à la pose d'implants dentaires ou auditifs.

Les implants dentaires sont posés pour l'heure de quatre façons :
1. En main libre avec une large découpe de la peau ou de la gencive et du périoste et leur décollement. Le forage se fait d'une manière archaïque sans aucun repère dans l'espace par rapport à la future prothèse. Bien que cette technique soit la plus mauvaise et donne des résultats souvent esthétiquement, fonctionnellement et hygiéniquement désastreux, elle est la plus utilisée. C'est également celle qui provoque le plus d'accidents (rupture du nerf mandibulaire, rupture d'artère, perçage du sinus, rupture des corticales, ...).
2. En main libre avec une large découpe de la gencive et du périoste et un décollement de ces derniers, le forage se fait d'une manière plus ou moins précise car le laboratoire dentaire a réalisé un guide chirurgical qui préfigure plus ou moins la future prothèse. Cette technique est la deuxième la plus utilisée, mais l'inconvénient est que le guide chirurgical ne contient aucune information de repérage dans l'espace. De plus, il est souvent inutilisable vu la découpe de la gencive qui empêche la mise en place de ce dernier. Avec cette technique les résultats sont souvent mauvais au niveau esthétique, fonctionnel, hygiénique et les accidents tels que précités sont nombreux.
3. En main guidée par des guides de forage réalisés à partir d'une planification informatisée. Cette technique permet de placer des cylindres de forage dans ces guides à des endroits précis en fonction de l'os ou en fonction de l'os et de la future prothèse. Trois technologies distinctes appliquent cette technique :
   - Des guides sont réalisés à partir de modèles stéréolitographiques, c'est-à-dire sur base d'images issues d'un balayage dentaire sous forme de DICOM. Des artéfacts perturbent souvent la réalisation de ces guides en ne permettant pas toujours de les utiliser par manque de précision.
   - Un guide est réalisé à partir d'une empreinte et d'un guide radiologique qui est réalisé sur base de cette empreinte en silicone. Un balayage du patient avec le guide en bouche et du guide seul est effectué. Un guide chirurgical est alors fabriqué à partir des images DICOM, et transformé par l'insertion de cylindres de guidage pour le forage et la pose d'implants dans la mâchoire. Des artéfacts perturbent ici aussi la réalisation de ces guides.
   - Des guides radiologiques transformés ensuite en guides chirurgicaux sont réalisés comme décrit dans la demande de brevet internationale antérieurement déposée PCT/EP2006/050584.

   Ces techniques permettent de réduire les dommages au patient et en particulier la dernière technique améliore le résultat prothétique.
4. En main libre guidée par un système de navigation (GPS). Cette technique permet de placer avec plus ou moins de précision un implant. Mais elle ne permet pas de prévenir tout dommage au patient car le forage reste manuel et un dérapage reste possible. De plus elle ne tient pas compte de la future prothèse. Cette technique est chère et est la moins utilisée.

Toutes ces techniques, à l'exception de celle où un balayage est fait le guide en bouche du patient et un balayage séparé du guide et celle décrite dans la demande de brevet PCT/EP2006/050584, présentent l'inconvénient de devoir réaliser la prothèse définitive après une prise d'empreinte de la mâchoire où les implants ont été placés préalablement, laquelle prise d'empreinte est réalisée plusieurs semaines après la pose des implants, ce qui est complexe et demande de nombreuses interventions post-opératoires, lourdes pour le patient.

Par ailleurs, certaines des techniques précitées prévoient le forage, que ce soit dans un modèle ou dans la mâchoire du patient, au travers de cylindres de guidage qui guident le foret pendant sa pénétration. Cette opération présente quelques inconvénients majeurs, entre autres la possibilité d'un blocage de la partie guidante du foret dans le cylindre si l'axe d'insertion est mal choisi, un freinage du moteur de l'appareil de forage par frottement contre la paroi interne du cylindre de guidage, une usure prématurée du moteur, une usure du cylindre de guidage avec risque de dispersion de particules métalliques dans le site opératoire, un échauffement du foret ou encore du cylindre de guidage avec possibilité de brûlure au niveau osseux.

La présente invention a pour but de mettre au point un procédé et un appareillage de fabrication d'une prothèse osseuse qui puisse être réalisée de manière définitive sur modèle, avant même la pose des implants, et de mettre en place cette prothèse sur les implants, le jour de la pose de ceux-ci dans l'os du patient, notamment comme décrit dans la demande de brevet précitée PCT/EP2006/050584, et cela en évitant simultanément les inconvénients indiqués ci-dessus.

Pour résoudre ce problème, on a prévu suivant l'invention un procédé de fabrication d'une prothèse osseuse à implanter dans un os d'un patient, comprenant
- une réalisation, à partir d'une empreinte de peau ou muqueuses du patient, d'un guide chirurgical doté d'au moins une prothèse artificielle et d'au moins un premier trou traversant chacun une prothèse artificielle susdite, suivant une orientation prédéterminée,
- un placement du guide chirurgical sur un modèle obtenu à partir de ladite empreinte,
- un forage à travers le modèle d'un deuxième trou à l'aide d'un foret passé au travers de chaque premier trou, le deuxième trou présentant l'orientation prédéterminée de son premier trou correspondant,
- un placement, dans chaque deuxième trou foré, d'un analogue d'implant présentant des dimensions correspondant à celles d'un implant réel à placer dans l'os du patient, ce placement étant effectué par l'intermédiaire d'un porte-analogue passé au travers du premier trou correspondant,
- une fixation de l'analogue d'implant dans son deuxième trou, tel que placé, et
- après enlèvement de chaque porte-analogue et du guide chirurgical, une confection d'une prothèse osseuse définitive adaptée au modèle pourvu du ou des analogues d'implant et destinée à être fixée sur un ou des implants réels après leur implantation dans l'os du patient à l'aide du guide chirurgical d'une manière semblable à celle utilisée pour le placement des analogues d'implant dans le modèle,
Ce procédé est caractérisé en ce qu'il comprend en outre
- pendant le forage d'un deuxième trou, un passage du foret et/ou d'un élément de prolongation de celui-ci au travers du premier trou correspondant sans contact avec celui-ci et
- un guidage du foret suivant ladite orientation prédéterminée par des moyens d'introduction de foret situés à l'extérieur dudit premier trou.

On a donc dissocié suivant l'invention la fonction de forage et celle de guidage, ce qui permet d'éviter dans le premier trou des frottements ou blocages entre le foret ou son élément de prolongation qui tournent sur eux-mêmes à grande vitesse, ainsi que l'usure des pièces qui découle de ces frottements.

Suivant un mode de réalisation avantageux de l'invention ledit guidage du foret est effectué par coopération de premiers moyens de guidage agencés sur le guide chirurgical à l'extérieur dudit au moins un premier trou et de seconds moyens de guidage reliés au foret.

Un guide chirurgical doté d'au moins une prothèse artificielle et d'au moins un premier trou traversant chacun une prothèse artificielle suivant une orientation prédéterminée peut être réalisé selon diverses méthodes connues en soi. On peut citer par exemple le procédé de réalisation d'un tel guide, décrit dans la demande de brevet PCT/EP2006/050584, WO 2006/082198, ou encore les techniques de Stéréolitographie, de fraisage numérique et de prototypage rapide bien connues de l'homme de métier. Avantageusement, pendant la réalisation du guide chirurgical, le procédé comprend un placement dans celui-ci d'au moins un élément creux, chaque élément creux étant pourvu d'un premier trou susdit.

La présente invention concerne également un appareillage destiné à une implantation de prothèse osseuse dans un os d'un patient, comprenant
- au moins un modèle réalisé à partir d'une empreinte de peau ou muqueuses du patient,
- un guide chirurgical, réalisé à partir de cette empreinte et doté d'au moins une prothèse artificielle et d'au moins un premier trou traversant chacun une prothèse artificielle susdite, suivant une orientation prédéterminée, et présentant un diamètre interne,
- au moins un premier foret qui présente un diamètre externe et qui, lorsque le guide chirurgical est en place sur le modèle, est capable de forer à travers le modèle un deuxième trou suivant une orientation identique à celle de chaque premier trou susdit, en passant à travers celui-ci,
- un analogue d'implant à loger dans chaque deuxième trou foré du modèle,
- un porte-analogue capable de porter de manière détachable un analogue d'implant susdit et de le loger dans son deuxième trou par un coulissement dans le premier trou correspondant, chaque porte-analogue étant muni de moyens de butée capables d'arrêter son coulissement lorsque l'analogue d'implant dans le deuxième trou est dans une position correspondant à une position chirurgicalement appropriée,
- une prothèse osseuse confectionnée sur le modèle libéré du ou des porte-analogue ainsi que du guide chirurgical,
- au moins un deuxième foret qui présente un diamètre externe et qui, lorsque le guide chirurgical est en place sur l'os du patient, est capable de passer à travers lesdits premiers trous et de forer des troisièmes trous dans l'os du patient suivant ladite orientation prédéterminée, ledit au moins un deuxième foret comportant des moyens de retenue capables d'arrêter une pénétration du deuxième foret à une profondeur prédéterminée,
- un implant à loger dans chaque troisième trou foré de l'os, et
- un porte-implant capable de porter de manière détachable un implant susdit et de le loger dans son troisième trou par un coulissement axial dans le premier trou correspondant, chaque porte-implant étant muni de moyens d'arrêt capables d'arrêter son coulissement, lorsque l'implant dans le troisième trou est dans une position chirurgicalement appropriée dans laquelle les implants sont capables de recevoir ladite prothèse osseuse préalablement réalisée sur le modèle.
Cet appareillage est caractérisé en ce que
le diamètre interne de chaque premier trou susdit est supérieur au diamètre externe dudit au moins un premier foret et dudit au moins un deuxième foret de façon qu'il n'y ait pas contact entre le foret et/ou un élément de prolongation de celui-ci et le premier trou à travers lequel il est passé pour effectuer un forage, et
l'appareillage comprend en outre des moyens d'introduction de foret dans chaque premier trou qui sont extérieurs à chaque premier trou et qui guident le foret parallèlement audit premier trou, suivant l'orientation prédéterminée.

Suivant une forme de réalisation de l'appareillage selon l'invention, lesdits moyens d'introduction de foret comportent des premiers moyens de guidage agencés sur le guide chirurgical à l'extérieur dudit au moins un premier trou et des seconds moyens de guidage reliés à chaque premier ou second foret et capables de coopérer avec les premiers moyens de guidage pour guider le foret suivant ladite orientation prédéterminée pendant le forage. Avantageusement, les premiers moyens de guidage consistent en au moins une perforation de guidage qui est prévue dans le guide chirurgical et qui s'étend parallèlement à chaque premier trou et les seconds moyens de guidage consistent en au moins une tige de guidage reliée au premier ou second foret, parallèlement à celui-ci et capable de pénétrer dans une perforation de guidage susdite pour permettre un guidage du premier ou second foret pendant le forage suivant l'orientation prédéterminée.

Suivant une forme perfectionnée de réalisation, lesdits premiers trous sont formés des éléments creux placés dans le guide chirurgical et présentant chacun une cavité axiale qui s'étend suivant ladite orientation prédéterminée. De préférence, les éléments creux sont pourvus d'au moins une perforation de guidage qui s'étend parallèlement à ladite cavité axiale.

D'autres particularités du procédé et de l'appareillage suivant l'invention sont indiquées dans les revendications annexées.

D'autres détails de l'invention ressortiront de la description donnée ci-après, à titre non limitatif et avec référence aux dessins annexés.
Les figures 1 à 5 représentent une vue en coupe d'un modèle pendant les étapes d'implantation dans celui-ci d'analogues d'implant et de réalisation de la prothèse définitive sur les analogues d'implant.
La figure 6 représente une vue en plan du dessus d'un élément creux à placer dans un guide chirurgical de l'appareillage suivant l'invention.
La figure 7 représente un contrangle muni d'un foret et de moyens de guidage suivant l'invention.
Les figures 8 et 9 représentent dans une vue en perspective une variante d'élément creux suivant l'invention et son élément porteur.

Sur les différentes figures, les éléments identiques ou analogues portent les mêmes références.

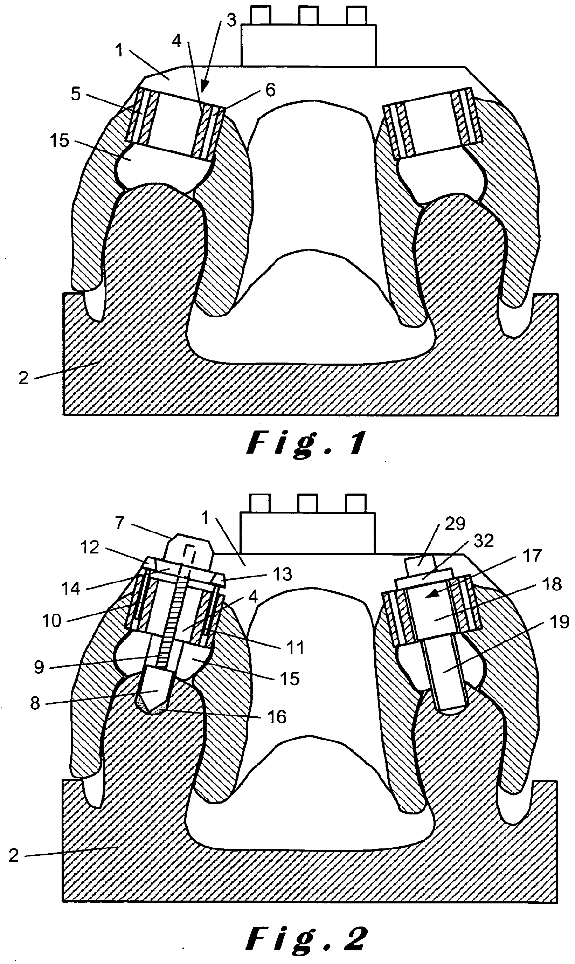

Sur la figure 1 on a représenté un guide chirurgical 1 en place sur un modèle 2 réalisé à partir de l'empreinte de la mâchoire du patient.

Dans l'exemple illustré le guide chirurgical 1 a été réalisé selon la technique expliquée dans la demande de brevet PCT/EP2006/050584. Comme on l'a déjà mentionné, ce guide pourrait être réalisé par d'autres techniques. Pour réaliser le guide chirurgical illustré, on a donc procédé à un façonnage sur le modèle d'un montage muni de fausses dents adaptables en bouche, à une réalisation d'une clef du montage, à un coulage, après enlèvement des fausses dents, dans la clef montée sur le modèle d'une matière visible en imagerie radiologique, à un durcissement de celle-ci sous la forme d'un arc, à une division de l'arc durci en dents artificielles radiologiques individuelles qui, replacées dans la clef, sont fixées sur le modèle et, après enlèvement de la clef, à la réalisation d'un guide radiologique par dépôt d'une résine autodurcissante sur les dents artificielles radiologiques fixées au modèle, ce guide radiologique étant muni de repères radiologiques. Ensuite, par traitement par ordinateur d'une image radiologique en deux dimensions représentant le guide radiologique en position sur la mâchoire, on constitue une image en trois dimensions. Dans ces images en deux et trois dimensions, on insère ensuite, par dent, un implant virtuel en position chirurgicalement appropriée dans l'image de la mâchoire et on place un élément creux virtuel présentant une cavité coaxiale à l'implant virtuel dans l'image du guide radiologique. A partir des données récoltées et calculées par l'ordinateur pendant ces étapes de traitement d'image, on réalise un guide chirurgical en forant dans le guide radiologique susdit et à travers chaque dent artificielle 15 un perçage approprié pour recevoir un élément creux réel 3 pourvu d'une cavité axiale 4 formant un premier trou et orientée suivant une direction prédéterminée, celle de l'implant virtuel dans l'image du guide radiologique.

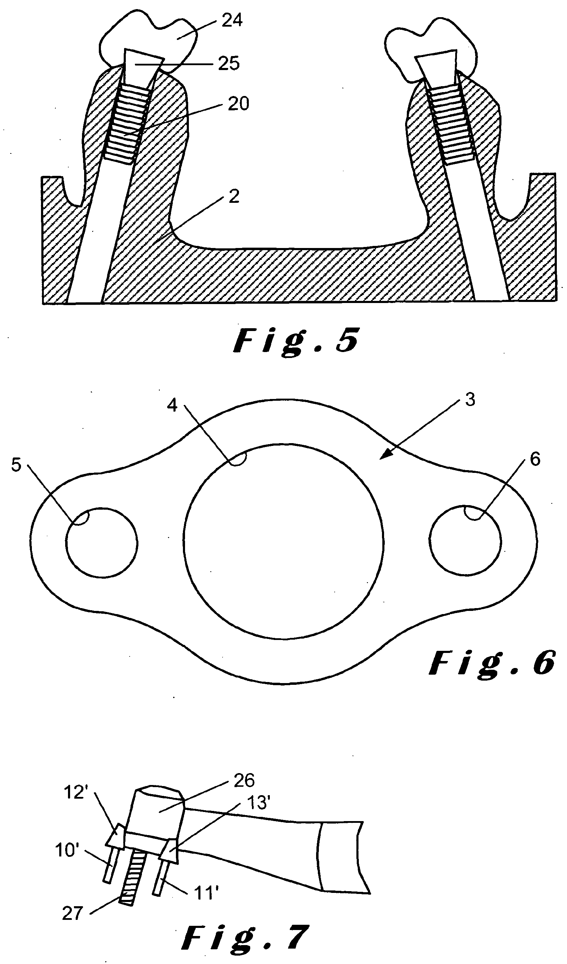

Dans l'exemple illustré en particulier sur les figures 1 et 6, l'élément creux 3 présente de part et d'autre de la cavité axiale 4 des perforations de guidage 5 et 6 qui sont situées à l'extérieur de la cavité axiale 4 et s'étendent parallèlement à celle-ci.

D'autre part, ainsi qu'il ressort de la figure 2 un appareil de forage 7 représenté uniquement de manière schématique est pourvu d'un foret 8 qui est passé au travers de la cavité axiale 4 et qui peut être entraîné en rotation par un moteur non représenté de l'appareil de forage, par l'intermédiaire d'une tige de support 9 qui sert d'élément de prolongation du foret 8.

L'appareillage de forage 7, et donc le foret 8, sont aussi dotés, dans l'exemple illustré, de deux tiges de guidage 10 et 11 qui s'étendent parallèlement au foret et sont capables de pénétrer dans les perforations de guidage 5 et 6. Ces tiges sont surmontées de butées 12 et 13 présentant un diamètre supérieur au diamètre interne des perforations 10 et 11. Elles sont reliées avantageusement à l'appareil de forage 7 et à la tige de support 9 par une bride 14 de manière que la tige de support 9 puisse tourner autour de son axe et que l'appareil de forage 7 puisse effectuer une rotation de préférence de 360° autour de la tige de support, alors que les tiges de guidage 10 et 11 ont déjà été introduites au moins partiellement dans les perforations de guidage 5 et 6.

Il doit être entendu que, si l'on applique d'autres techniques de fabrication de guide chirurgical que celle utilisée pour l'exemple illustré, il est possible de façonner le premier trou correspondant à la cavité axiale 4 et les perforations de guidage 5 et 6 directement dans la masse du guide chirurgical 1, sans devoir placer d'élément creux 3 dans celle-ci.

En fonction de la profondeur de forage souhaitée, la longueur du foret sera choisie en tenant compte à la fois de la longueur de guidance des tiges 10 et 11 jusqu'à ce que les butées 12 et 13 arrêtent la course du foret 8 et de la profondeur à laquelle l'élément de prolongation du foret est introduite dans l'appareil de forage 7.

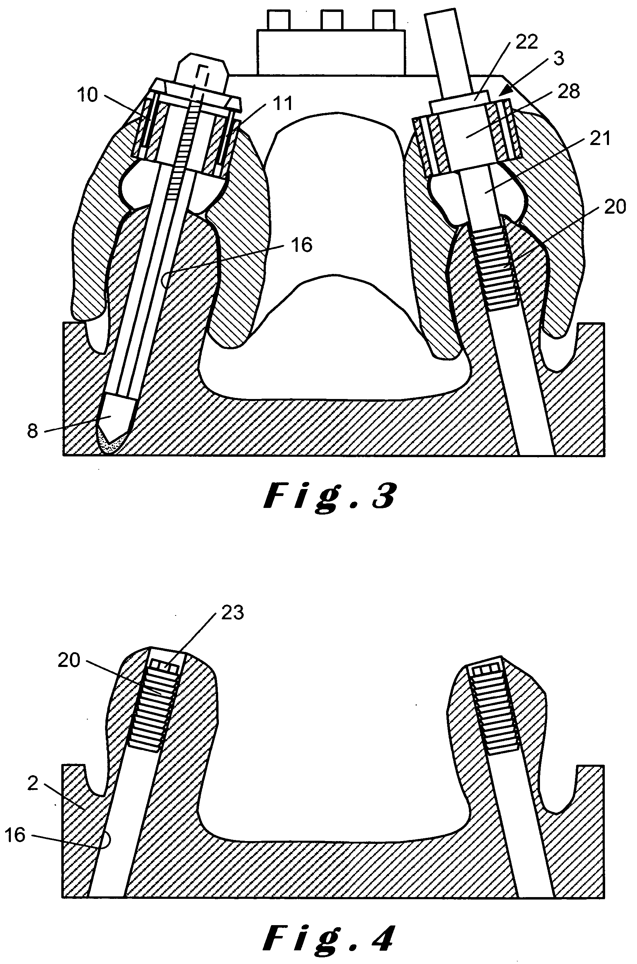

Dans l'exemple illustré sur les figures 2 et 3, le forage est effectué en plusieurs étapes. Dans la partie gauche de la figure 2, un foret 8 est passé à travers la cavité axiale 4 ou premier trou du guide chirurgical, sans entrer en contact avec les parois de cette cavité. Le foret 8 et sa tige de prolongation 9 présentent en effet un diamètre externe inférieur au diamètre interne de la cavité axiale 4. De plus, les tiges de guidage 10 et 11 qui pénètrent dans les perforations de guidage 5 et 6 guident le foret suivant l'orientation prédéterminée de la cavité, c'est-à-dire axialement. Tout contact entre foret 8 et élément creux 3 est ainsi évité pendant le forage, ce qui permet de surmonter les inconvénients de la technique antérieure. Il faut remarquer qu'une seule perforation de guidage et une unique tige de guidage correspondante suffiraient déjà. Au cours du forage le foret 8 fore un deuxième trou 16 dans le modèle 2, jusqu'à ce que les butées 12 et 13 arrêtent toute pénétration supplémentaire.

On peut alors retirer l'appareil de forage 7 et extraire le foret 8 hors de l'élément creux 3. Avant de commencer à forer un nouveau deuxième trou dans le modèle, on introduit dans le deuxième trou 16 foré dans l'étape de forage initiale un élément de maintien en place 17 qui assure la position du guide chirurgical 1 sur le modèle 3 pendant qu'on fore un autre deuxième trou. Cet élément de maintien en place 17 est illustré, en service, sur la partie de droite de la figure 2.

Cet élément de maintien en place 17 comporte, au centre, un corps cylindrique lisse 18 qui est adapté aux dimensions de la cavité axiale 4 de l'élément creux. A une extrémité ce corps cylindrique est prolongé par une tige 19 adaptée aux dimensions du deuxième trou 16 et à l'extrémité opposée il est pourvu d'une bride 32 de diamètre plus large que le premier trou formé par la cavité axiale 4 et d'une poignée 29. Cet élément est ainsi capable de bloquer en place le guide chirurgical sur le modèle pendant qu'on fore un deuxième trou 16 voisin.

Quand tous les deuxièmes trous ont été forés à une première profondeur, on peut poursuivre le forage à une seconde profondeur. Dans ce cas on ôte l'élément de maintien en place 17 d'un élément creux 3, en laissant en place les autres. On introduit alors un deuxième foret dont la tige de prolongation 9 est plus longue, et on fore de la même manière que décrit précédemment, c'est-à-dire de façon guidée par les tiges de guidage 10 et 11 et les perforations de guidage 5 et 6. On peut ainsi en arriver à la profondeur atteinte sur la partie gauche de la figure 3 et traverser complètement le modèle.

Comme illustré sur la partie droite de la figure 3, on peut alors introduire dans le deuxième trou 16 un analogue d'implant 20 présentant des dimensions correspondant à celles d'un implant réel à placer dans la mâchoire du patient. Ce placement est effectué par l'intermédiaire d'une porte-analogue 21 qui comporte une partie cylindrique 28 capable de coulisser de manière guidée dans la cavité axiale 4 de l'élément creux 3, et qui est capable d'amener l'analogue d'implant 20 à la profondeur requise dans le deuxième trou 16. Le porte-analogue 21 présente à cet effet une bride 22 qui arrête le coulissement de l'analogue d'implant 20 dans le deuxième trou 16 correspondant. Le porte-analogue peut éventuellement aussi amener à tourner l'analogue d'implant 20 dans une position angulaire appropriée, ce qui peut être nécessaire, lorsque celui-ci porte, comme illustré sur la figure 4, une tête 23 de section polygonale.

On peut alors fixer en position l'analogue d'implant dans le trou 16 par exemple par une colle appropriée que l'on introduit dans le trou 16 par l'envers du modèle 2. On peut alors enlever le porte-analogue (voir figure 4). Le modèle est prêt pour la réalisation d'une simulation préimplantaire sous la forme d'une prothèse définitive, par exemple des barres, des couronnes, ou des bridges. Une prothèse en position sur le modèle est illustrée sur la figure 5 où un élément d'aboutement 25 a été fixé sur chaque analogue d'implant avec pardessus la dent artificielle définitive correspondante 24.

Ensuite, le guide chirurgical 1, muni de ses premiers trous et perforations de guidage correspondantes, c'est-à-dire dans l'exemple illustré des éléments creux 3, est placé dans la bouche du patient.

On fait alors usage d'un appareil de forage approprié, connu en soi, par exemple un contrangle 26. Celui-ci présente un foret 27, présentant un diamètre inférieur au diamètre interne de la cavité axiale 4. Comme l'appareil de forage 7, le contrangle 26 est équipé de tiges de guidage 10' et 11' qui peuvent coulisser dans les perforations de guidage 5 et 6 des éléments creux. Les butées 12' et 13' arrêtent le forage à une profondeur prédéterminée, qui ici aussi peut s'effectuer en plusieurs étapes. Ici aussi il est préférable de forer chaque troisième trou dans la mâchoire à une première profondeur avant d'en forer un à une profondeur plus importante. Pendant que l'on fore dans un troisième trou, les autres reçoivent un élément de maintien en place 17 qui assure la position du guide chirurgical sur la mâchoire. Lors du dernier forage, les forets, en plus d'être guidés dans une orientation correcte par les perforations et tiges de guidage, sont arrêtés dans leur coulissement par les butées 12', 13' que portent les tiges de guidage à une hauteur appropriée correspondant à la profondeur de l'implant sur les images en deux et trois dimensions.

Dans chacun de ces trous, on introduit alors un implant semblable à l'analogue d'implant à l'aide d'un porte-implant semblable au porte-analogue, c'est-à-dire pourvu d'un moyen d'arrêt de coulissement, sous la forme par exemple d'une bride. L'implant est ainsi enfoncé à la profondeur requise dans l'orientation appropriée. A l'aide d'un repère le porte-implant peut éventuellement amener en rotation la tête de l'implant de section identique à celle de l'analogue d'implant à la même position angulaire que la tête de l'analogue d'implant sur laquelle la prothèse a été façonnée.

La position des implants dans la bouche est unique et elle correspond parfaitement à celle des analogues d'implant dans le modèle, ainsi qu'à celle apparaissant sur les images radiologiques en deux et trois dimensions. On peut donc y placer immédiatement la prothèse qui a été fabriquée avant la pose des implants.

Un avantage de cette technique est aussi que grâce à l'anticipation prothétique on connaît les positions relatives exactes des implants / prothèse / os et que toutes les sécurités de profondeur, de rotation et de positions latérales sont déterminées et figées. Ainsi toutes erreurs humaines par forage sont exclues.

L'appareillage suivant l'invention permet en outre un guidage parfait du contrangle sans que le foret ne risque un blocage ou un frottement sur les parois de la cavité axiale des éléments creux. On élimine ainsi tout risque de fracture de la partie interne du contrangle et de pénétration de particules métalliques dans le site d'implantation. Enfin il ne se produit plus d'échauffement du foret par frottement entre la partie guidante du foret et la cavité axiale de l'élément creux. Une brûlure au niveau osseux est ainsi évitée.

En outre les fluides d'irrigation et de refroidissement du foret ne sont plus bloqués par la partie guidante du foret.

Il doit être entendu que la présente invention n'est en aucune façon limitée aux formes de réalisation décrites ci-dessus et que bien des modifications peuvent y être apportées sans sortir du cadre des revendications annexées.

On peut par exemple prévoir des tiges de guidage 10 et 11 de plus grande longueur que celles prévues dans l'exemple illustré, notamment pour pouvoir réaliser un forage en moins d'étapes, ou même en une seule étape. Dans ce cas, lors de la mise au point du guide chirurgical 1, on réalise le perçage des dents artificielles 15 de manière appropriée non seulement pour recevoir l'élément creux 3, mais aussi pour pouvoir introduire les tiges de guidage 10 et 11 lorsqu'elles dépassent l'élément creux 3 après avoir été passées au travers des perforations de guidage 5, 6 de celui-ci.

On peut même prévoir aussi, pendant le traitement par ordinateur de l'image radiologique du guide radiologique en position sur la mâchoire, d'insérer dans l'image en 2D ou 3D des tiges de guidage virtuelles permettant de contrôler de manière correspondante le perçage prédécrit du guide radiologique en guide chirurgical capable de recevoir des tiges de guidage réelles.

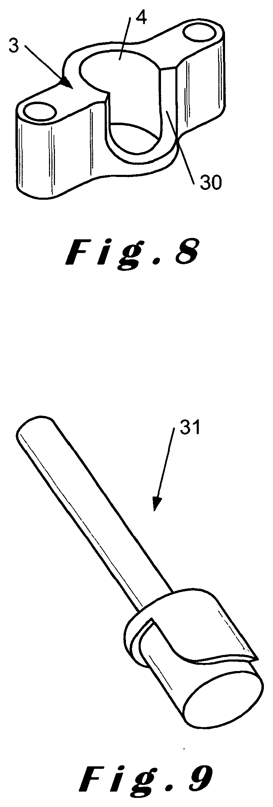

Sur la figure 8, on a par ailleurs représenté un élément creux adapté pour être placé sur le guide chirurgical à des emplacements difficiles d'accès, par exemple les parties arrière des mâchoires. Par rapport à l'élément creux représenté sur la figure 6, celui-ci présente un évidement latéral 30 qui permet un accès radial à la cavité axiale 4. Cet agencement facilite la pénétration du contrangle et de son foret dans l'élément creux par introduction radiale, puis pivotement à 90°.

Sur la figure 9, on a représenté le porte-élément creux 31 correspondant qui permet de placer l'élément creux dans le guide chirurgical.

## Revendications

1. Procédé de fabrication d'une prothèse osseuse à implanter dans un os d'un patient, comprenant
- une réalisation, à partir d'une empreinte de peau ou de muqueuses du patient, d'un guide chirurgical doté d'au moins une prothèse artificielle et d'au moins un premier trou traversant chacun une prothèse artificielle susdite, suivant une orientation prédéterminée,
- un placement du guide chirurgical sur un modèle obtenu à partir de ladite empreinte,
- un forage à travers le modèle d'un deuxième trou à l'aide d'un foret passé au travers de chaque premier trou, le deuxième trou présentant l'orientation prédéterminée de son premier trou correspondant,
- un placement, dans chaque deuxième trou foré, d'un analogue d'implant présentant des dimensions correspondant à celles d'un implant réel à placer dans l'os du patient, ce placement étant effectué par l'intermédiaire d'un porte-analogue passé au travers du premier trou correspondant,
- une fixation de l'analogue d'implant dans son deuxième trou, tel que placé, et
- après enlèvement de chaque porte-analogue et du guide chirurgical, une confection d'une prothèse osseuse définitive adaptée au modèle pourvu du ou des analogues d'implant et destinée à être fixée sur un ou des implants réels après leur implantation dans l'os du patient à l'aide du guide chirurgical d'une manière semblable à celle utilisée pour le placement des analogues d'implant dans le modèle,
**caractérisé en ce qu'**il comprend en outre
- pendant le forage d'un deuxième trou, un passage du foret et/ou d'un élément de prolongation de celui-ci au travers du premier trou correspondant sans contact avec celui-ci et
- un guidage du foret suivant ladite orientation prédéterminée par des moyens d'introduction de foret situés à l'extérieur dudit premier trou.

2. Procédé suivant la revendication 1, **caractérisé en ce que** ledit guidage du foret est effectué par coopération de premiers moyens de guidage agencés sur le guide chirurgical à l'extérieur dudit au moins un premier trou et de seconds moyens de guidage reliés au foret.

3. Procédé suivant l'une des revendications 1 et 2, **caractérisé en ce que**, en présence de plusieurs deuxièmes trous à forer, leur forage est effectué en plusieurs étapes correspondant à plusieurs profondeurs de forage, chaque deuxième trou étant foré à une première profondeur avant qu'un deuxième trou ne soit foré à une deuxième profondeur supérieure à la première, et **en ce que** le procédé comprend, après forage d'un deuxième trou à une première profondeur, une introduction d'un élément de maintien du guide chirurgical dans ce deuxième trou et le premier trou correspondant pour assurer en place le guide chirurgical pendant un forage dans un autre deuxième trou et ainsi de suite.

4. Procédé suivant l'une des revendications 1 à 3, **caractérisé en ce que**, pendant la réalisation du guide chirurgical, il comprend un placement dans celui-ci d'au moins un élément creux, chaque élément creux étant pourvu d'un premier trou susdit.

5. Appareillage destiné à une implantation de prothèse osseuse dans un os d'un patient, comprenant
- au moins un modèle (2) réalisé à partir d'une empreinte de peau ou de muqueuses du patient,
- un guide chirurgical (1), réalisé à partir de cette empreinte et doté d'au moins une prothèse artificielle (15) et d'au moins un premier trou (4) traversant chacun une prothèse artificielle susdite, suivant une orientation prédéterminée, et présentant un diamètre interne,
- au moins un premier foret (8) qui présente un diamètre externe et qui, lorsque le guide chirurgical (1) est en place sur le modèle (2), est capable de forer à travers le modèle un deuxième trou (16) suivant une orientation identique à celle de chaque premier trou (4) susdit, en passant à travers celui-ci,
- un analogue d'implant (20) à loger dans chaque deuxième trou (16) foré du modèle (2),
- un porte-analogue (21) capable de porter de manière détachable un analogue d'implant (20) susdit et de le loger dans son deuxième trou (16) par un coulissement dans le premier trou correspondant, chaque porte-analogue (21) étant muni de moyens de butée (22) capables d'arrêter son coulissement lorsque l'analogue d'implant (20) dans le deuxième trou (16) est dans une position correspondant à une position chirurgicalement appropriée,
- une prothèse osseuse (24, 25) confectionnée sur le modèle (2) libéré du ou des porte-analogue (21) ainsi que du guide chirurgical (1),
- au moins un deuxième foret qui présente un diamètre externe et qui, lorsque le guide chirurgical (1) est en place sur l'os du patient, est capable de passer à travers lesdits premiers trous (4) et de forer des troisièmes trous dans l'os du patient suivant ladite orientation prédéterminée, ledit au moins un deuxième foret comportant des moyens de retenue capables d'arrêter une pénétration du deuxième foret à une profondeur prédéterminée,
- un implant à loger dans chaque troisième trou foré de l'os, et
- un porte-implant capable de porter de manière détachable un implant susdit et de le loger dans son troisième trou par un coulissement axial dans le premier trou correspondant, chaque porte-implant étant muni de moyens d'arrêt capables d'arrêter son coulissement, lorsque l'implant dans le troisième trou est dans une position chirurgicalement appropriée dans laquelle les implants sont capables de recevoir ladite prothèse osseuse (24, 25) préalablement réalisée sur le modèle (2),
**caractérisé en ce que**
le diamètre interne de chaque premier trou (4) susdit est supérieur au diamètre externe dudit au moins un premier foret (8) et dudit au moins un deuxième foret de façon qu'il n'y ait pas contact entre le foret et/ou un élément de prolongation (9, 27) de celui-ci et le premier trou (4) à travers lequel il est passé pour effectuer un forage, et
l'appareillage comprend en outre des moyens d'introduction de foret (5, 6, 10, 11) dans chaque premier trou qui sont extérieurs à chaque premier trou (4) et qui guident le foret (8) parallèlement audit premier trou (4), suivant l'orientation prédéterminée.

6. Appareillage suivant la revendication 5, **caractérisé en ce que** lesdits moyens d'introduction de foret comportent des premiers moyens de guidage (5, 6) agencés sur le guide chirurgical (1) à l'extérieur dudit au moins un premier trou (4) et des seconds moyens de guidage (10, 11) reliés à chaque premier ou second foret et capables de coopérer avec les premiers moyens de guidage (5, 6) pour guider le foret suivant ladite orientation prédéterminée pendant le forage.

7. Appareillage suivant la revendication 6, **caractérisé en ce que** les premiers moyens de guidage consistent en au moins une perforation de guidage (5, 6) qui est prévue dans le guide chirurgical (1) et qui s'étend parallèlement à chaque premier trou (4) et **en ce que** les seconds moyens de guidage consistent en au moins une tige de guidage (10, 11) reliée au premier ou second foret, parallèlement à celui-ci et capable de pénétrer dans une perforation de guidage (5, 6) susdite pour permettre un guidage du premier ou second foret pendant le forage suivant l'orientation prédéterminée.

8. Appareillage suivant l'une ou l'autre des revendications 5 à 7, **caractérisé en ce que** lesdits premiers trous sont formés par des éléments creux (3) placés dans le guide chirurgical (1) et présentant chacun une cavité axiale (4) qui s'étend suivant ladite orientation prédéterminée.

9. Appareillage suivant la revendication 8, **caractérisé en ce que** les éléments creux (3) sont pourvus d'au moins une perforation de guidage (10, 11) qui s'étend parallèlement à ladite cavité axiale (4).

10. Appareillage suivant l'une des revendications 8 et 9, **caractérisé en ce que** chaque porte-analogue (21) ou porte-implant comporte une partie cylindrique (27) capable de coulisser de manière guidée dans la cavité axiale (4) des éléments creux (3).

11. Appareillage suivant l'une quelconque des revendications 8 à 10, **caractérisé en ce qu'**au moins un élément creux (3) présente un évidement latéral (30) qui permet un accès radial dans la cavité axiale (4).

12. Appareillage suivant l'une quelconque des revendications 5 à 11, **caractérisé en ce qu'**il comprend en outre au moins un élément de maintien en place (17) du guide chirurgical (1) sur le modèle (2) ou sur l'os qui est à enfoncer dans un premier trou (4) et une partie d'un second (16) ou troisième trou correspondant après un forage d'au moins cette partie du second ou troisième trou correspondant et avant forage d'un autre second ou troisième trou.

13. Procédé suivant la revendication 4, **caractérisé en ce que** l'élément creux comprend une cavité axiale (4) formant un premier trou susdit et, de part et d'autre de la cavité axiale, des perforations de guidage (5 et 6) qui sont situées à l'extérieur de la cavité axiale (4) et s'étendent parallèlement à celle-ci.

14. Procédé suivant la revendication 13, **caractérisé en ce que** l'élément creux présente en outre un évidement latéral (30) qui permet un accès radial dans la cavité axiale (4).

## Patentansprüche

1. Herstellungsverfahren einer Knochenprothese zur Implantation in einen Knochen eines Patienten, umfassend
- Fertigen, aus einem Haut- oder Schleimhautabdruck des Patienten, einer Operationsschablone, die mit mindestens einer künstlichen Prothese und mindestens einem ersten Loch versehen ist, das in einer vorgegebenen Ausrichtung jeweils durch eine vorgenannte künstliche Prothese verläuft,
- Platzieren der Operationsschablone auf einem Modell, das aus dem Abdruck erhalten worden ist,
- Bohren eines zweiten Lochs durch das Modell mit einem durch jedes erste Loch geführten Bohrer, wobei das zweite Loch die vorgegebene Ausrichtung seines entsprechenden ersten Lochs aufweist,
- Platzieren eines Manipulierimplantats in jedem gebohrten zweiten Loch, das Abmessungen aufweist, die denen eines echten, in den Knochen des Patienten einzubringenden Implantats entsprechen, wobei dieses Platzieren mit einem Manipulierimplantatträger erfolgt, der durch das erste entsprechende Loch geführt wird,
- Befestigen des Manipulierimplantats in seinem zweiten Loch im platzierten Zustand, und
- nach dem Entfernen jedes Manipulierimplantatträgers und der Operationsschablone Anfertigen einer endgültigen Knochenprothese, die an das Modell angepasst ist, das mit dem Manipulierimplantat oder den Manipulierimplantaten versehen ist, und die an einem echten Implantat oder echten Implantaten nach ihrer Implantation im Knochen des Patienten mithilfe der Operationsschablone auf ähnliche Weise wie bei der Platzierung der Manipulierimplantate im Modell befestigt werden soll,
**dadurch gekennzeichnet, dass** es ferner Folgendes umfasst:
- während des Bohrens eines zweiten Lochs Hindurchfiihren des Bohrers und/oder eines Verlängerungselements davon durch das entsprechende erste Loch ohne Berührung desselben, und
- Führen des Bohrers in der vorgegebenen Ausrichtung mit Mitteln zum Einführen des Bohrers, die sich außerhalb des ersten Lochs befinden.

2. Verfahren nach Anspruch 1, **dadurch gekennzeichnet, dass** das Führen des Bohrers durch ein Zusammenwirken erster Führungsmittel, die an der Operationsschablone außerhalb des mindestens einen ersten Lochs angeordnet sind, und zweiter Führungsmittel erfolgt, die mit dem Bohrer verbunden sind.

3. Verfahren nach einem der Ansprüche 1 und 2, **dadurch gekennzeichnet, dass** bei Vorhandensein von mehreren zweiten zu bohrenden Löchern sie in mehreren Schritten gebohrt werden, die mehreren Bohrtiefen entsprechen, wobei jedes zweite Loch in einer ersten Tiefe gebohrt wird, bevor ein zweites Loch in einer zweiten Tiefe gebohrt wird, die tiefer als die erste ist, und dadurch, dass das Verfahren nach dem Bohren eines zweiten Lochs in einer ersten Tiefe ein Einbringen eines Elements zum Halten der Operationsschablone in diesem zweiten Loch und dem entsprechenden ersten Loch umfasst, damit sichergestellt ist, dass die Operationsschablone während des Bohrens in einem weiteren zweiten Loch an der richtigen Stelle bleibt, und so weiter.

4. Verfahren nach einem der Ansprüche 1 bis 3, **dadurch gekennzeichnet, dass** es während des Fertigens der Operationsschablone ein Platzieren von mindestens einem Hohlelement in derselben umfasst, wobei jedes Element mit einem vorgenannten ersten Loch versehen ist.

5. Vorrichtung zum Implantieren einer Knochenprothese in einen Knochen eines Patienten, umfassend
- mindestens ein Modell (2), das aus einem Haut- oder Schleimhautabdruck des Patienten gefertigt ist,
- eine Operationsschablone (1), die aus diesem Abdruck gefertigt ist und mit mindestens einer künstlichen Prothese (15) und mindestens einem ersten Loch (4) versehen ist, das in einer vorgegebenen Ausrichtung jeweils durch eine vorgenannte künstliche Prothese verläuft und einen Innendurchmesser aufweist,
- mindestens einen ersten Bohrer (8), der einen Außendurchmesser aufweist und der, wenn sich die Operationsschablone (1) an der richtigen Stelle am Modell (2) befindet, durch das Modell ein zweites Loch (16) in einer Ausrichtung bohren kann, die mit der jedes vorgenannten ersten Lochs (4) genau übereinstimmt, und dabei dort hindurch geführt wird,
- ein Manipulierimplantat (20), das in jedem gebohrten zweiten Loch (16) des Modells (2) eingesetzt werden soll,
- einen Manipulierimplantatträger (21), der ein vorgenanntes Manipulierimplantat (20) lösbar tragen kann und es durch Schieben in das entsprechende erste Loch in seinem zweiten Loch (16) einsetzen kann, wobei jeder Manipulierimplantatträger (21) mit Anschlagmitteln (22) versehen ist, die in der Lage sind, seine Verschiebung zu stoppen, wenn sich das Manipulierimplantat (20) im zweiten Loch (16) in einer Position befindet, die einer für die Operation geeigneten Position entspricht,
- eine auf dem Modell (2) erstellte Knochenprothese (24, 25) ohne den oder die Manipulierimplantatträger (21) sowie die Operationsschablone (1),
- mindestens einen zweiten Bohrer, der einen Außendurchmesser aufweist und der, wenn sich die Operationsschablone (1) an der richtigen Stelle am Knochen des Patienten befindet, in der Lage ist, durch die ersten Löcher (4) hindurchgeführt zu werden und dritte Löcher in den Knochen des Patienten in der vorgegebenen Ausrichtung zu bohren, wobei der mindestens eine zweite Bohrer Rückhaltemittel aufweist, die in der Lage sind, eine Durchdringung des zweiten Bohrers in einer vorgegebenen Tiefe zu stoppen,
- ein in jedes gebohrte dritte Loch des Knochens einzusetzendes Implantat, und
- einen Implantatträger, der lösbar ein vorgenanntes Implantat tragen kann und es durch axiales Schieben in das entsprechende erste Loch in sein drittes Loch einsetzen kann, wobei jeder Implantatträger mit Anhaltemitteln versehen ist, die in der Lage sind, seine Verschiebung zu stoppen, wenn sich das Implantat im dritten Loch in einer für die Operation geeigneten Position befindet, in der die Implantate die zuvor auf dem Modell (2) gefertigte Knochenprothese (24, 25) aufnehmen können,
**dadurch gekennzeichnet, dass**
der Innendurchmesser jedes vorgenannten ersten Lochs (4) größer ist als der Außendurchmesser des mindestens einen ersten Bohrers (8) und des mindestens einen zweiten Bohrers, sodass sich der Bohrer und/oder ein Verlängerungselement (9, 27) desselben und das erste Loch (4), durch das er geführt wird, damit eine Bohrung erfolgt, nicht berühren, und
die Vorrichtung ferner Mittel zum Einführen des Bohrers (5, 6, 10, 11) in jedes erste Loch umfasst, die sich außerhalb jedes ersten Lochs (4) befinden und die den Bohrer (8) parallel zum ersten Loch (4) in der vorgegebenen Ausrichtung führen.

6. Vorrichtung nach Anspruch 5, **dadurch gekennzeichnet, dass** die Mittel zum Einführen des Bohrers erste Führungsmittel (5, 6) aufweisen, die an der Operationsschablone (1) außerhalb des mindestens einen ersten Lochs (4) angeordnet sind, und zweite Führungsmittel (10, 11), die mit jedem ersten oder zweiten Bohrer verbunden und in der Lage sind, mit den ersten Führungsmitteln (5, 6) zusammenzuwirken, um den Bohrer während des Bohrens in der vorgegebenen Ausrichtung zu führen.

7. Vorrichtung nach Anspruch 6, **dadurch gekennzeichnet, dass** die ersten Führungsmittel aus mindestens einem Führungsloch (5, 6) bestehen, das in der Operationsschablone (1) vorgesehen ist und parallel zu jedem ersten Loch (4) verläuft, und dadurch, dass die zweiten Führungsmittel aus mindestens einer Führungsstange (10, 11) bestehen, die mit dem ersten oder zweiten Bohrer parallel dazu verbunden und in der Lage ist, ein vorgenanntes Führungsloch (5, 6) zu durchqueren, damit der erste oder zweite Bohrer während des Bohrens in der vorgegebenen Ausrichtung geführt werden kann.

8. Vorrichtung nach einem der Ansprüche 5 bis 7, **dadurch gekennzeichnet, dass** die ersten Löcher von Hohlelementen (3) gebildet sind, die in der Operationsschablone (1) angeordnet sind und jeweils einen axialen Hohlraum (4) aufweisen, der in der vorgegebenen Ausrichtung verläuft.

9. Vorrichtung nach Anspruch 8, **dadurch gekennzeichnet, dass** die Hohlelemente (3) mit mindestens einem Führungsloch (10, 11) versehen sind, das parallel zu dem axialen Hohlraum (4) verläuft.

10. Vorrichtung nach einem der Ansprüche 8 und 9, **dadurch gekennzeichnet, dass** jeder Manipulierimplantatträger (21) oder Implantatträger einen zylindrischen Teil (27) aufweist, der in der Lage ist, geführt in dem axialen Hohlraum (4) der Hohlelemente (3) verschoben zu werden.

11. Vorrichtung nach einem der Ansprüche 8 bis 10, **dadurch gekennzeichnet, dass** mindestens ein Hohlelement (3) eine seitliche Aussparung (30) aufweist, die einen radialen Zugang zu dem axialen Hohlraum (4) gewährt.

12. Vorrichtung nach einem der Ansprüche 5 bis 11, **dadurch gekennzeichnet, dass** sie ferner mindestens ein Lagefixierungselement (17) für die Operationsschablone (1) an dem Modell (2) oder an dem Knochen umfasst, das in ein erstes Loch (4) und einen Teil eines entsprechenden zweiten (16) oder dritten Lochs gesteckt werden soll, nachdem zumindest dieser Teil des entsprechenden zweiten oder dritten Lochs gebohrt wurde und bevor ein weiteres zweites oder drittes Loch gebohrt ist.

13. Verfahren nach Anspruch 4, **dadurch gekennzeichnet, dass** das Hohlelement einen axialen Hohlraum (4) umfasst, der ein vorgenanntes erstes Loch bildet, und, auf beiden Seiten des axialen Hohlraums, Führungslöcher (5 und 6), die sich außerhalb des axialen Hohlraums (4) befinden und parallel dazu verlaufen.

14. Verfahren nach Anspruch 13, **dadurch gekennzeichnet, dass** das Hohlelement ferner eine seitliche Aussparung (30) aufweist, die einen radialen Zugang zu dem axialen Hohlraum (4) gewährt.

## Claims

1. A method of producing a bone prosthesis to be implanted in a patient's bone, comprising:
- production, from an impression of the skin or mucous membranes of the patient, of a surgical guide equipped with at least one artificial prosthesis and at least one first hole, each passing through an aforementioned artificial prosthesis in a predetermined direction;
- placement of the surgical guide on a model obtained from said impression;
- drilling through the model of a second hole using a drill passed through each first hole, the second hole having the predetermined direction of its corresponding first hole;
- placement, in each drilled second hole, of an implant analog having dimensions corresponding to those of a real implant to be placed in the patient's bone, this placement being carried out by means of an analog holder passed through the corresponding first hole;
- fixing the implant analog in its second hole, as placed; and
- after removal of each analog holder and of the surgical guide, construction of a final bone prosthesis matching the model provided with the implant analog(s) and intended to be fixed to one or more real implants after their implantation in the patient's bone using the surgical guide in a similar manner to that used for placing implant analogs in the model,
**characterized in that** it furthermore comprises:
- during the drilling of a second hole, passage of the drill and/or an extension element extending this drill through the corresponding first hole without contact with it; and
- guiding the drill in said predetermined direction using means for inserting the drill situated outside said first hole.

2. The method as claimed in claim 1, **characterized in that** said guiding of the drill is effected through cooperation of first guiding means, arranged on the surgical guide outside said at least one first hole, with second guiding means connected to the drill.

3. The method as claimed in either of claims 1 and 2, **characterized in that**, in the presence of several second holes to be drilled, their drilling is carried out in several steps corresponding to several drilling depths, each second hole being drilled to a first depth before a second hole is drilled at a second depth greater than the first, and **in that** the method comprises, after drilling a second hole to a first depth, insertion of an element for holding the surgical guide in this second hole and the corresponding first hole in order to ensure the placing of the surgical guide during drilling in another second hole, and so on.

4. The method as claimed in any one of claims 1 to 3, **characterized in that**, during production of the surgical guide, it comprises a placement therein of at least one hollow element, each hollow element being provided with an aforementioned first hole.

5. Equipment intended for an implantation of a bone prosthesis in a bone of a patient, comprising:
- at least one model (2) produced from an impression of the skin or mucous membranes of the patient;
- a surgical guide (1), produced from this impression and equipped with at least one artificial prosthesis (15) and at least one first hole (4), each passing through an aforementioned artificial prosthesis in a predetermined direction and having an internal diameter;
- at least one first drill (8) that has an external diameter and which, when the surgical guide (1) is in place on the model (2), is capable of drilling in a second hole (16) through the model in an identical direction to that of each aforementioned first hole (4), while passing through this;
- an implant analog (20) to be housed in each drilled second hole (16) in the model (2);
- an analog holder (21) capable of holding, in a detachable manner, an aforementioned implant analog (20) and of housing it in its second hole (16) by sliding in the corresponding first hole, each analog holder (21) being equipped with stop means (22) capable of stopping it sliding when the implant analog (20) in the second hole (16) is in a position corresponding to a surgically appropriate position;
- a bone prosthesis (24, 25) constructed on the model (2) freed of the analog holder(s) (21) and of the surgical guide (1);
- at least one second drill which has an external diameter and which, when the surgical guide (1) is in place on the patient's bone, is capable of passing through said first holes (4) and of drilling third holes in the patient's bone in said predetermined direction, said at least one second drill comprising retaining means capable of stopping penetration of the second drill at a predetermined depth;
- an implant to be housed in each third hole drilled in the bone; and
- an implant holder capable of holding, in a detachable manner, an aforementioned implant and of housing it in its third hole, by axially sliding into the corresponding first hole, each implant holder being equipped with stopping means capable of stopping it sliding when the implant in the third hole is in a surgically appropriate position in which the implants are capable of receiving said bone prosthesis (24, 25) previously produced on the model (2);
**characterized in that**
- the internal diameter of each aforementioned first hole (4) is greater than the external diameter of said at least one first drill (8) and said at least one second drill so that there is no contact between the drill and/or an extension element (9, 27) thereof and the first hole (4) through which it is passed to carry out a drilling; and
- the equipment furthermore comprises means for inserting the drill (5, 6, 10, 11) into each first hole that are external to each first hole (4) and which guide the drill (8) parallel to said first hole (4) in the predetermined direction.

6. The equipment as claimed in claim 5, **characterized in that** said means for inserting the drill comprise first guiding means (5, 6) arranged on the surgical guide (1) outside said at least one first hole (4) and second guiding means (10, 11) connected to each first or second drill and capable of cooperating with the first guiding means (5, 6) in order to guide the drill in said predetermined direction during the drilling.

7. The equipment as claimed in claim 6, **characterized in that** the first guiding means consist of at least one guiding hole (5, 6), which is provided in the surgical guide (1) and which extends parallel to each first hole (4) and **in that** the second guiding means consist of at least one guiding rod (10, 11) connected to the first or second drill, parallel to this and capable of penetrating into an aforementioned guiding hole (5, 6) in order to enable guiding of the first or second drill while drilling in the predetermined direction.

8. The equipment as claimed in any one of claims 5 to 7, **characterized in that** said first holes are formed from hollow elements (3) placed in the surgical guide (1) and each having an axial cavity (4) that extends in said predetermined direction.

9. The equipment as claimed in claim 8, **characterized in that** the hollow elements (3) are provided with at least one guiding hole (10, 11) that extends parallel to said axial cavity (4).

10. The equipment as claimed in either of claims 8 and 9, **characterized in that** each analog holder (21) or implant holder comprises a cylindrical part (27) capable of sliding in a guided manner in the axial cavity (4) of the hollow elements (3).

11. The equipment as claimed in any one of claims 8 to 10, **characterized in that** at least one hollow element (3) has a lateral recess (30) which enables radial access into the axial cavity (4).

12. The equipment as claimed in any one of claims 5 to 11, **characterized in that** it furthermore comprises at least one placeholding element (17) for holding the surgical guide (1) in place on the model (2) or on the bone which is to be set in a first hole (4) and a part of a corresponding second (16) or third hole after a drilling of at least this part of the corresponding second or third hole and before drilling of another second or third hole.

13. The method as claimed in claim 4, **characterized in that** the hollow element comprises an axial cavity (4) forming an aforementioned first hole and, on both sides of the axial cavity, guiding holes (5 and 6) that are situated outside the axial cavity (4) and extend parallel to this.

14. The method as claimed in claim 13, **characterized in that** the hollow element furthermore has a lateral recess (30) which permits radial access into the axial cavity.
